# EUROPÄISCHE PATENTANMELDUNG

(11) **EP 0 870 836 A1**
(43) Veröffentlichungstag der Anmeldung: **14.10.1998**
(21) Anmeldenummer: 97105855.7
(22) Anmeldetag: 09.04.1997
(51) Int. Cl.: C12N 15/82, C12N 9/16

(54) **2-Desoxyglucose-6-Phosphat (2-DOG-6-P) Phosphatase DNA-Sequenzen als Selektionsmarker in Pflanzen**

(71) Anmelder: IPK Gatersleben, 06466 Gatersleben (DE)
(72) Erfinder: Sonnewald, Uwe, 06484 Quedlinburg (DE); Ebneth, Marcus, 12163 Berlin (DE)
(74) Vertreter: VOSSIUS & PARTNER

(57) **Zusammenfassung**

Es werden rekombinante DNA-Moleküle beschrieben, die eine DNA-Sequenz enthalten, die ein Protein mit der biologischen Aktivität einer 2-Desoxyglucose-6-Phosphat (2-DOG-6-P) Phosphatase codiert und die unter der Kontrolle von regulatorischen Sequenzen eines in Pflanzen aktiven Promotors und Transkriptions-Terminations und/oder Polyadenylierungssignalen steht. Weiterhin werden Vektoren und Wirtszellen beschrieben, die die erfindungsgemäßen rekombinanten DNA-Moleküle enthalten. Ferner werden Verfahren zur Herstellung transformierter Pflanzenzellen und Pflanzen unter Verwendung der beschriebenen rekombinanten DNA-Moleküle und Vektoren bereitgestellt. Desweiteren werden transgene Pflanzen, deren Ernteprodukte und Vermehrungsmaterial sowie Pflanzenzellen und -Gewebe beschrieben, die die erfindungsgemäßen rekombinanten DNA-Moleküle oder Vektoren enthalten oder durch das erfindungsgemäße Verfahren hergestellt werden.

## Beschreibung

Die vorliegende Erfindung betrifft die Verwendung von DNA-Sequenzen, die ein Protein mit der biologischen Aktivität einer 2-Desoxyglucose-6-Phosphat (2-DOG-6-P) Phosphatase codieren, als Selektionsmarker in Pflanzenzellen zur Selektion transformierter Pflanzen. Weiterhin betrifft diese Erfindung rekombinante DNA-Moleküle, die derartige DNA-Sequenzen enthalten, wobei diese mit regulatorischen Sequenzen eines in Pflanzen aktiven Promotors und Transkriptions-Terminations und/oder Polyadenylierungssignalen operativ verbunden sind. Ferner werden Vektoren, Wirtszellen und Kits beschrieben, die derartige rekombinante DNA-Moleküle enthalten, sowie mit den rekombinanten DNA-Molekülen transformierte Pflanzenzeilen und Pflanzen. Die vorliegenden Erfindung betrifft ebenfalls Verfahren zur Herstellung transgener Pflanzen, die aufgrund der Einführung der oben beschriebenen rekombinanten DNA-Moleküle auf 2-Desoxyglucose enthaltenden Medien selektioniert werden können. Schließlich betrifft die vorliegende Erfindung transgene Pflanzen, Pflanzenzellen und Gewebe, die das erfindungsgemäße DNA-Molekül enthalten oder durch das vorstehend beschriebene Verfahren erhalten werden, sowie Ernteprodukte und Vermehrungsmaterial der beschriebenen transgenen Pflanzen.

Mittels gentechnischer Verfahren ist es möglich, gezielt Fremdgene in das pflanzliche Genom zu übertragen. Dieser Prozeß wird als Transformation und die resultierenden Pflanzen als transgen bezeichnet. Die vornehmlichen Ziele sind zum einen der Pflanzenschutz und zum anderen eine Qualitätssteigerung der Ernteprodukte. Beispiele für Pflanzenschutzmaßnahmen sind: (i) Herbizidtolerante Pflanzen (DE-A-3701623; Stalker (1988) Science 242, 419), (ii) Insektenresistente Pflanzen (Vaek (1987) Plant Cell 5, 159-169), (iii) Virusresistente Pflanzen (Powell (1986) Science 232, 738-743) und (vi) Ozon-resistente Pflanzen Van Camp (1994) BioTech. 12, 165-168). Beispiele für Qualitätssteigerungen sind: (i) Erhöhung der Haltbarkeit von Früchten (Oeller (1991) Science 254, 437-439), (ii) Erhöhung der Stärkeproduktion in Kartoffelknollen (Stark (1992) Science 242, 419), (iii) Veränderung der Stärke- (Visser (1991) Mol. Gen. Genet. 225, 289-296) und Lipidzusammensetzung (Voelker (1992) Science 257, 72-74) und (iv) Produktion Pflanzenfremder Polymere (Poirer (1992) Science 256, 520-523).
Grundvoraussetzung für die Erzeugung transgener Pflanzen ist die Verfügbarkeit geeigneter Transformationssysteme und das Vorhandensein selektionierbarer Marker, die die Identifizierung erfolgreich transformierter Pflanzenzellen ermöglichen.
Zur Transformation stehen derzeit mehrere Verfahren zur Verfügung. Die am häufigsten eingesetzte Methode zur Tranformation dikotyledoner Pflanzen ist der Agrobacterium-vermittelte Gentransfer. Hierbei wird die natürliche Fähigkeit des Bodenbakteriums ausgenutzt, genetisches Material in das pflanzliche Genom zu integrieren. Weitere geeignete Verfahren sind beispielsweise Protoplasten-Transformation durch Polyethylenglykol-induzierte DNA-Aufnahme, Elektroporation, Sonikation oder Mikroinjektion sowie die Transformation intakter Zellen oder Gewebe durch Mikro- oder Makroinjektion in Gewebe oder Embryonen, Gewebeelektroporation, Inkubation trockener Embryonen in DNA-haltiger Lösung, Vakuuminfiltration von Samen und biololistischer Gentransfer.
Da unabhängig vom Transformationsverfahren nur wenige Zellen die gewünschten Eigenschaften tragen, wird in herkömmlicher Weise neben dem Zielgen ein selektionierbarer Marker in das pflanzliche Genom integriert, der die Identifizierung transgener Zellen ermöglicht. Derzeit werden zur Selektion transformierter Pflanzenzellen vornehmliche Gene eingesetzt, die eine Herbizid- oder Antibiotikatoleranz vermitteln. Geeignete Resistenzgene sind beispielsweise das bar-Gen aus Streptomyces hygroscopicus, das Resistenz gegen das Totalherbizid Phosphinothricin vermittelt (De Block (1987) EMBO J. 6, 2513-2518) oder das nptII-Gen aus dem Transposon Tn5 von Escherichia coli, das Resistenz gegen das Antibiotikum Kanamycin bewirkt (Herrera-Estrella (1983) EMBO J. 2, 987-995). Je nach Pflanzenart, sind die genannten Verfahren nicht immer effektiv und beeinflussen häufig die Pflanzenregeneration negativ. Darüber hinaus ist der Einsatz von Genen, die Antibiotikaresistenzen vermitteln, im Lebensmittelbereich unerwünscht. Desweiteren ist zur Steuerung komplexer Stoffwechselprozessee die Manipulation mehrerer enzymatischer Schritte notwendig. D.h. die Möglichkeit transgene Pflanzen mehrfach zu transformieren ist im Bereich des "Metabolic Engineering" essentiell. Die genannten Gründe haben dazu geführt, daß die Suche nach weiteren selektionierbaren Markern verstärkt vorangetrieben wird. Trotz intensiver Bemühungen sind nur wenige neue Marker zur Selektion transformierter Pflanzenzellen erfolgreich eingesetzt worden. Durch Expression einer Mannose-6-Phosphat Isomerase konnte eine positive Selektion auf Mannose-haltigen Nährmedien für transformierte Pflanzenzellen etabliert werden (WO 94/20627). Ein weiteres Verfahren nutzt die Fähigkeit einer Deaminase aus Aspergillus terreus aus, das Insektizid Blasticidin S zu detoxifizieren (Tamura (1995) Biosci. Biotechnol. Biochem. 59, 2336-2338).

Der vorliegenden Erfindung liegt somit die Aufgabe zugrunde, rekombinante DNA-Moleküle zur Verfügung zu stellen, die eine DNA-Sequenz enthalten, die zur Selektion transformierter Pflanzenzellen oder Pflanzen eingesetzt werden kann.
Diese Aufgabe wird durch die Bereitstellung der in den Patentansprüchen gekennzeichneten Ausführungsform gelöst.

Somit betrifft die vorliegenden Erfindung ein rekombinantes DNA-Molekül umfassend
(a) regulatorische Sequenzen eines in Pflanzen aktiven Promotors;
(b) operativ daran gebunden eine DNA-Sequenz, die ein Protein mit der biologischen Aktivität einer 2-Desoxyglucose-6-Phosphat (2-DOG-6-P) Phosphatase codiert; und
(c) operativ daran gebunden regulatorische Sequenzen, die als Transkriptions-Terminations- und/oder Polyadenylierungs-signale in Pflanzen dienen können.

Im Zusammenhang mit der vorliegenden Erfindung wird unter einem Protein mit der biologischen Aktivität einer 2-DOG-6-P Phosphatase ein Protein verstanden, das in der Lage ist, nicht-metabolisierbare glucoseanaloge Verbindungen wie 2-DOG in nicht-toxische Produkte umzusetzen. Die toxische Wirkung von 2-DOG entsteht nach Phosphorylierung zum 2-DOG-6-P, d.h. die Phosphatase hebt die Wirkung von 2-DOG-6-P durch Dephosphorylierung auf. Ein alternativer Resistenzmechanismus, der ebenfalls im Sinne der vorliegenden Erfindung genutzt werden kann, besteht darin, die Phosphorylierung oder die Aufnahme von 2-DOG zu unterbinden.
Entsprechende Mutanten wurden in Hefe beschrieben, z.B. eine Transportmutante (Novak (1990) FEBS Lett. 269, 202-204) und eine Phosphorylierungsmutante (Lobo (1977) Mol. Gen. Genet. 157, 297-300).

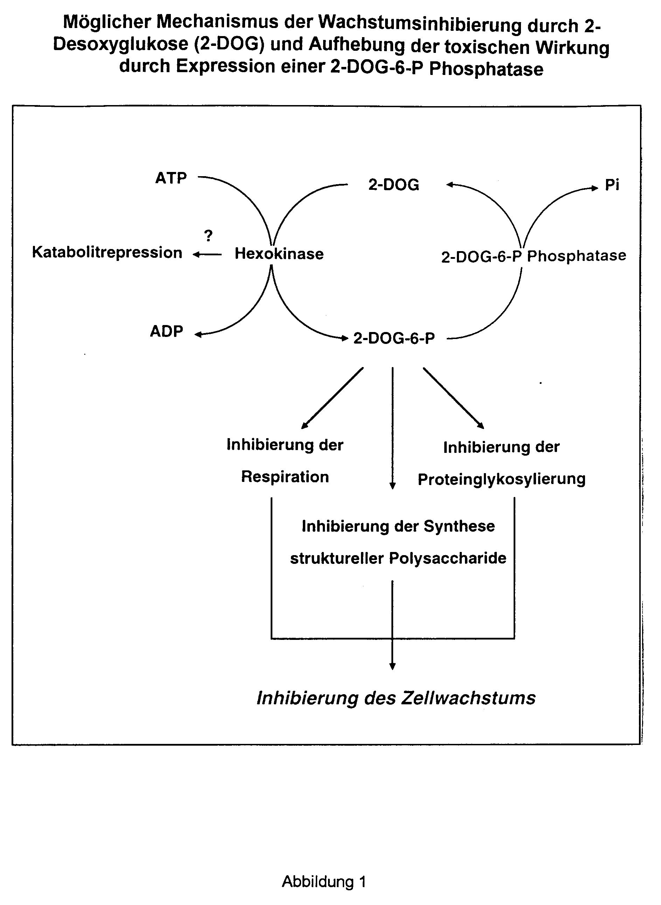

Überraschenderweise wurde gefunden, daß die Expression einer 2-DOG-6-P Phosphatase aus Hefe Resistenz gegenüber 2-DOG vermitteln und zur Selektion transformierter Pflanzen eingesetzt werden kann, die ansonsten phänotypisch normal und fertil sind. Aus Untersuchungen in Hefe war bekannt, daß Zugabe von 2-DOG, einem nicht-metabolisierbaren Glucoseanalogon, zur Inhibierung der Respiration und des Wachstums von Zellen führt (Heredia (1964) Biochem.
Biophys. Acta 86, 216). In Hefezellen korreliert die Wachstumsinhibierung mit einer verminderten Synthese struktureller Polysaccharide (Kratky (1975) Eur. J. Biochem. 54, 459) und einer Blockierung der Proteinglykosylierung (Datema & Schwarz (1978) Eur. J. Biochem. 90, 505), die vermutlich durch Veränderungen der Zuckernucleotidkonzentrationen hervorgerufen werden. Über die biochemischen Ånderungen hinaus führt die Zugabe von 2-DOG zu einer Reprimierung einer Vielzahl von Genen (zusammengefasst in, Gancedo (1992) Eur. J. Biochem. 206, 297). Analog zur Wirkungsweise metabolisierbarer Zucker (wie z.B. Glucose), wird dieser Prozeß als Katabolitrepression bezeichnet.
Untersuchungen an Hefezellen haben gezeigt, daß der 2-DOG Wirkmechanismus von der intrazellulären 2-DOG-6-P Konzentration abhängig ist. Molekulare Charakterisierung von Hefemutanten, die resistent gegenüber 2-DOG sind, ergab, daß die erworbene Resistenz auf der Überexpression einer spezifischen 2-DOG-6-P Phosphatase beruht (Sanz (1994) Yeast 10, 1195). Die hypothetische Wirkungsweise sowie der Wirkmechanismus der 2-DOG-6-P Phosphatase ist in Abbildung 1 dargestellt.
Im Gegensatz zu den zahlreichen Arbeiten über die Wirkungsweise von 2-DOG in tierischen Geweben, in Hefen und Bakterien gibt es allerdings nur wenig analoge Studien, die sich mit dem Stoffwechsel dieser Zucker in Pflanzen befassen. Bisher konnte in einigen Untersuchungen lediglich gezeigt werden, daß Zugabe von 2-DOG das Wurzelwachstum einer Vielzahl von Pflanzen hemmt (u.a. Flachs, Wicke, Klee, Roggen, Gerste, Mais, Hafer und Gerste (Stenlid (1959) Physiol. Plant. 12, 218; Farrar (1995) J. Exp. Bot. 46, 1859)). Darüber hinaus wird das Wachstum von Nicotiana tabacum-Zellen und Picea excelsa (Fichte)-Zellen in Gewebekultur durch 2-DOG stark gehemmt (Zemek (1975) Z. Pflanzenphysiol. 76, 114; Zemek (1976) Z. Pflanzenphysiol. 77, 95). Als Umsetzungsprodukte von 2-DOG in höheren Pflanzen konnte 2-DOG-1-P, 2-DOG-6-P, UDP-2-DOG sowie 2-DOG enthaltende Di- und Oligosaccharide nachgewiesen werden (Kocourek (1963) Biochem. Biophys. Acta 71, 497; Zemek (1975) Z. Pflanzenphysiol. 76, 114). Die Bildung dieser Stoffwechselprodukte wird für die inhibitorische Wirkung der 2-DOG verantwortlich gemacht. Für andere toxische Verbindungen, die als Selektionsmarker in der Pflanzentransformation eingesetzt werden, sind ebenfalls vielfältige Wirkungen auf den Metabolismus der Pflanze beschrieben. So führt z. B. die Zugabe von manchen Antibiotika oftmals zur Regeneration von transgenen Pflanzen, die physiologische und morphologische Veränderungen gegenüber dem Phänotyp des Wildtyps aufweisen und/oder steril sind.
Obwohl wie vorstehend erläutert auch 2-DOG eine Vielzahl von teilweise noch unbekannten biochemischen Änderungen in Zellen hervorruft, werden bei der Verwendung der erfindungsgemäßen rekombinanten DNA-Moleküle in Verbindung mit der Selektion auf 2-DOG enthaltenen Medien phänotypisch normale und fertile transgene Pflanzen regeneriert.

Die DNA-Sequenz, die ein Protein mit der biologischen Aktivität einer 2-DOG-6-P Phosphatase codiert, kann aus natürlichen Quellen isoliert werden, vorzugsweise Hefe, oder kann nach bekannten Verfahren synthetisiert werden.
Mittels gängiger molekularbiologischer Techniken ist es möglich (siehe z.B. Sambrook, 1989, Molecular Cloning, A Laboratory Manual, 2. Aufl. Cold Spring Harbor Laboratory Press, Cold Spring Harbor, NY), verschiedenartige Mutationen in die DNA-Sequenz, die ein Protein mit der biologischen Aktivität einer 2-DOG-6-P Phosphatase codiert, in den erfindungsgemäßen rekombinanten DNA-Molekülen einzuführen, wodurch es zur Synthese von Proteinen mit eventuell veränderten biologischen Eigenschaften kommt. Hierbei ist zum einen die Erzeugung von Deletionsmutanten möglich, bei denen durch fortschreitende Deletionen vom 5'- oder vom 3'- Ende der codierenden DNA-Sequenz, die Synthese entsprechend verkürzter Proteine erreicht werden kann. Ferner ist es möglich, gezielt Enzyme herzustellen, die durch Addition entsprechende Signalsequenzen in bestimmten Kompartimenten der Pflanzenzelle lokalisiert sind. Derartige Sequenzen sind bekannt (siehe beispielsweise Braun, EMBO J. 11 (1992), 3219-3227; Wolter, Proc. Natl. Acad. Sci. USA 85 (1988), 846-850; Sonnewald, Plant J. 1 (1991), 95-106).
Andererseits ist auch die Einführung von Punktmutationen denkbar an Positionen, bei denen eine Veränderung der Aminosäuresequenz einen Einfluß beispielsweise auf die Enzymaktivität oder die Regulierung des Enzyms hat. Auf diese Weise können z.B. Mutanten hergestellt werden, die einen veränderten Kₘ-Wert besitzen oder nicht mehr den normalerweise in der Zelle vorliegenden Regulationsmechanismen über allosterische Regulation oder kovalente Modifizierung unterliegen.
Des weiteren können Mutanten hergestellt werden, die eine veränderte Substrat- oder Produktspezifität aufweisen. Weiterhin können Mutanten hergestellt werden, die ein verändertes Aktivitäts-Temperatur-Profil aufweisen.
Für die gentechnische Manipulation in prokaryontischen Zellen können die erfindungsgemäßen rekombinanten DNA-Moleküle oder Teile dieser Moleküle in Plasmide eingebracht werden, die eine Mutagenese oder eine Sequenzveränderung durch Rekombination von DNA-Sequenzen erlauben. Mit Hilfe von Standardverfahren (vgl. Sambrook, 1989, Molecular Cloning: A laboratory manual, 2. Aufl., Cold Spring Harbor Laboratory Press, NY, USA) können Basenaustausche vorgenommen oder natürliche oder synthetische Sequenzen hinzugefügt werden.
Für die Verbindung der DNA-Fragmente untereinander können an die Fragmente Adaptoren oder Linker angesetzt werden. Ferner können Manipulationen, die passende Restriktionsschnittstellen zur Verfügung stellen oder die überflüssige DNA oder Restriktionsschnittstellen entfernen, eingesetzt werden. Wo Insertionen, Deletionen oder Substitutionen in Frage kommen, können in vitro-Mutagenese, "primer repair", Restriktion oder Ligation verwendet werden. Als Analysemethode werden im allgemeinen eine Sequenzanalyse, eine Restriktionsanalyse und weitere biochemisch-molekularbiologische Methoden durchgeführt.

In einer bevorzugten Ausführungsform ist die DNA-Sequenz, die ein Protein mit der biologischen Aktivität einer 2-DOG-6-P Phosphatase codiert, ausgewählt aus der Gruppe bestehend aus
(a) DNA-Sequenzen, die eine Nucleotid-Sequenz umfassen, die die in SEQ ID NO. 2 angegebene Aminosäuresequenz codieren;
(b) DNA-Sequenzen, die die in SEQ ID NO. 1 angegebene Nucleotidsequenz umfassen;
(c) DNA-Sequenzen umfassend eine Nucleotidsequenz, die mit einem komplementären Strang der Nucleotidsequenz von (a) oder (b) hybridisieren;
(d) DNA-Sequenzen umfassend eine Nucleotidsequenz, die zu einer Nucleotidsequenz von (c) degeneriert ist, und
(e) DNA-Sequenzen, die ein Derivat, Analog oder Fragment einer Nucleotidsequenz von (a), (b), (c) oder (d) ist und für ein Protein codiert das 2-DOG-6-P Phosphatase Aktivität besitzt.

Der Begriff "Hybridisierung" bedeutet im Rahmen dieser Erfindung eine Hybridisierung unter konventionellen Hybridisierungsbedingungen, vorzugsweise unter stringenten Bedingungen, wie sie beispielsweise in Sambrook (Molecular Cloning, A Laboratory Manual, 2. Aufl. (1989) Cold Spring Harbor Laboratory Press, Cold Spring Harbor, NY) beschrieben sind.
DNA-Sequenzen, die mit den DNA-Sequenzen, die ein Protein mit der biologischen Aktivität einer 2-DOG-6-P Phosphatase codieren, hybridisieren, können z.B. aus genomischen oder aus cDNA-Bibliotheken isoliert werden, die aus Hefe hergestellt wurden. Die Identifizierung und Isolierung derartiger DNA-Sequenzen kann dabei z.B. unter Verwendung der DNA-Sequenzen erfolgen, die exakt oder im wesentlichen die unter SEQ ID NO. 1 angegebene Nucleotidsequenz oder Teile dieser Sequenzen aufweisen bzw. die reverse Komplemente dieser DNA-Sequenzen, z.B. mittels Hybridisierung nach Standardverfahren (siehe z.B. Sambrook, 1989, Molecular Cloning, A Laboratory Manual, 2. Aufl. Cold Spring Harbor Laboratory Press, Cold Spring Harbor, NY). Bei den als Hybridisierungsprobe verwendeten Fragmenten kann es sich auch um synthetische Fragmente handeln, die mit Hilfe üblicher Synthesetechniken hergestellt wurden und deren Sequenz im wesentlichen mit der in SEQ ID NO. 2 dargestellten übereinstimmt. Die DNA-Sequenzen, die ein Protein mit der biologischen Aktivität einer 2-DOG-6-P Phosphatase codieren, umfassen auch DNA-Sequenzen, deren Nucleotidsequenzen zu der einer der vorstehend beschriebenen DNA-Sequenzen degeneriert ist. Die Degeneration des genetischen Codes bietet dem Fachmann u.a. die Möglichkeit, die Nucleotidsequenz der DNA-Sequenz an die Codon-Präferenz des jeweiligen Wirts, vorzugsweise Pflanzen, anzupassen.
Die oben beschriebenen DNA-Sequenzen umfassen auch Fragmente, Derivate und allelische Varianten der oben beschriebenen DNA-Sequenzen, die ein Protein mit der biologischen Aktivität einer 2-DOG-6-P Phosphatase codieren. Unter "Fragmenten" werden dabei Teile der DNA-Sequenz verstanden, die lang genug sind, um eines der beschriebenen Proteine zu codieren. Der Ausdruck "Derivat" bedeutet in diesem Zusammenhang, daß die Sequenzen sich von den oben beschriebenen DNA-Sequenzen an einer oder mehreren Positionen unterscheiden aber einen hohen Grad an Homologie zu diesen Sequenzen aufweisen. Homologie bedeutet dabei eine Sequenzidentität von mindestens 40 %, insbesondere eine Identität von mindestens 60 %, vorzugsweise über 80 % und besonders bevorzugt über 90 %. Dabei weisen die durch diese DNA-Sequenzen codierten Proteine eine Sequenzidentität zu der in SEQ ID NO. 2 angegebenen Aminosäuresequenz von mindestens 80%, vorzugsweise 85% und besonders bevorzugt über 90%, 95%, 97% und 99% auf. Die Abweichungen zu den oben beschriebenen DNA-Sequenzen können dabei beispielsweise durch Deletion, Substitution, Insertion oder Rekombination entstanden sein.
Bei den DNA-Sequenzen, die homolog zu den oben beschriebenen Sequenzen sind und Derivate dieser Sequenzen darstellen, handelt es sich in der Regel um Variationen dieser Sequenzen, die Modifikationen darstellen, die dieselbe biologische Funktion ausüben. Es kann sich dabei sowohl um natürlicherweise auftretende Variationen handeln, beispielsweise um Sequenzen aus anderen Organismen, oder um Mutationen, wobei diese Mutationen auf natürliche Weise aufgetreten sein können oder durch gezielte Mutagenese eingeführt wurden. Ferner kann es sich bei den Variationen um synthetisch hergestellte Sequenzen handeln.
Bei den allelischen Varianten kann es sich sowohl um natürlich auftretende Varianten handeln, als auch um synthetisch hergestellte oder durch rekombinante DNA-Techniken erzeugte Varianten.

Die von den verschiedenen Varianten der in den rekombinanten DNA-Molekülen enthaltenen DNA-Sequenzen codierten Proteine, die die biologische Aktivität einer 2-DOG-6-P Phosphatase besitzen, weisen bestimmte gemeinsame Charakteristika auf, wie Enzymaktivität, Molekulargewicht, immunologische Reaktivität oder Konformation oder physikalische Eigenschaften, wie das Laufverhalten in Gelelektrophoresen, chromatographisches Verhalten, Sedimentationskoeffizienten, Löslichkeit, spektroskopische Eigenschaften, Stabilität; pH-Optimum, Temperatur-Optimum.

In einer besonders bevorzugten Ausführungsform stammt die beschriebene DNA-Sequenz aus Hefe.

Zur Expression der in den erfindungsgemäßen rekombinanten DNA-Molekülen enthaltenen DNA-Sequenz in pflanzlichen Zellen ist diese mit regulatorischen Sequenzen verknüpft, die die Transkription in pflanzlichen Zellen gewährleisten.
Hierzu zählen insbesondere Promotoren. Generell kommt für die Expression jeder in pflanzlichen Zellen aktive Promotor in Frage.
Der Promotor kann dabei so gewählt sein, daß die Expression konstitutiv erfolgt oder nur in einem bestimmten Gewebe, zu einem bestimmten Zeitpunkt der Pflanzenentwicklung oder zu einem durch äußere Einflüsse determinierten Zeitpunkt. In Bezug auf die Pflanze kann der Promotor homolog oder heterolog sein.
Sinnvolle Promotoren sind z.B. der Promotor der 35S RNA des Cauliflower Mosaic Virus und der Ubiquitin-Promotor aus Mais für eine konstitutive Expression, besonders bevorzugt ein Promotor, der eine Expression lediglich in photosynthetisch aktiven Geweben sicherstellt, z.B. der ST-LS1-Promotor (Stockhaus, Proc. Natl. Acad. Sci. USA 84 (1987), 7943-7947; Stockhaus, EMBO J. 8 (1989), 2445-2451) oder ein Promotor, der während der Pflanzentransformation, der Pflanzenregeneration oder bestimmten Stadien dieser Prozesse aktiv ist, z.B. Zellteilungsspezifische Promotoren wie der Histon H-3 Promotor (Kapros (1993), In Vitro Cell Dev. Biol. Plant 29, 27-32) oder das chemisch-induzierbare Tet-System (Gatz (1991), Mol. Gen. Genet. 227, 229-237). Eine Übersicht weiterer in Frage kommender Promotoren ist z.B. in Ward (1993) Plant Mol. Biol. 22, 361-366 gegeben.

Weiterhin bevorzugt sind induzierbare oder Zell-spezifische Promotoren (z.B. Meristem).
Ferner ist eine Transkriptions-Terminationssequenz vorhanden, die der korrekten Beendigung der Transkription dient sowie der Addition eines Poly-A-Schwanzes an das Transkript dienen kann, dem eine Funktion bei der Stabilisierung der Transkripte beigemessen wird. Derartige Elemente, z.B. der Terminator des Octopinsynthasegens aus Agrobakterien, sind in der Literatur beschrieben (vgl. Gielen, EMBO J. 8 (1989), 23-29) und sind beliebig austauschbar.

In einer bevorzugten Ausführungsform ist der Promotor der 35S CAMV Promotor.

Die Erfindung betrifft ferner Vektoren, die erfindungsgemäße rekombinante DNA-Moleküle enthalten. Vorzugsweise handelt es sich dabei um Plasmide, Cosmide, Viren, Bacteriophagen und andere in der Gentechnik übliche Vektoren. Zur Vorbereitung der Einführung fremder Gene in höhere Pflanzen stehen eine große Anzahl von Clonierungsvektoren zur Verfügung, die ein Replikationssignal für E.coli und ein Markergen zur Selektion transformierter Bakterienzellen enthalten. Beispiele für derartige Vektoren sind pBR322, pUC-Serien, M13mp-Serien, pACYC184 usw.
Die gewünschte Sequenz kann an einer passenden Restriktionsschnittstelle in den Vektor eingeführt werden. Das erhaltene Plasmid wird für die Transformation von E.coli-Zellen verwendet. Transformierte E.coli-Zellen werden in einem geeigneten Medium gezüchtet, anschließend geerntet und lysiert. Das Plasmid wird wiedergewonnen. Als Analysemethode zur Charakterisierung der gewonnenen Plasmid-DNA werden im allgemeinen Restriktionsanalysen, Gelelektrophoresen und weitere biochemisch-molekularbiologische Methoden eingesetzt. Nach jeder Manipulation kann die Plasmid-DNA gespalten und gewonnene DNA-Fragmente mit anderen DNA-Sequenzen verknüpft werden. Jede Plasmid-DNA-Sequenz kann in den gleichen oder anderen Plasmiden cloniert werden.

In einer bevorzugten Ausführungsform enthält der erfindungsgemäße Vektor mindestens ein weiteres rekombinantes DNA-Molekül. Durch die Bereitstellung der erfindungsgemäßen DNA-Moleküle und Vektoren kann jede beliebige Information, die in einem weiteren rekombinanten DNA-Molekül enthalten ist, auf Pflanzen bzw. Pflanzenzellen übertragen werden und anschließend mittels Selektion auf 2-DOG enthaltenen Medien auf die gewünschte Information selektioniert werden. Natürlich weiß der Fachmann, daß das rekombinante DNA-Molekül, das die zusätzliche Information enthält, nicht unbedingt in dem Vektor, der den Selektionsmarker trägt, anwesend sein muß, sondern auch mit diesen co-transformiert werden kann (Lyznik (1989) Plant Mol. Biol. 13, 151-161; Peng (1995) Plant Mol. Biol. 27, 91-104). Diese Möglichkeit bietet sich zum Beispiel an, wenn keine physikalische Koppelung des Markergens und der zu übertragenden Information gewünscht wird. In diesem Fall können nach der Selektion der primären transgenen Pflanze das Markergen und die gewünschte Information in nachfolgenden Kreuzungen unabhängig voneinander seggregieren.

In einer besonders bevorzugten Ausführungsform enthält das weitere rekombinante DNA-Molekül eine DNA Sequenz, die ein Peptid, Protein, Antisense-, Sense-RNA, virale RNA, oder Ribozym codiert. Einige Beispiele zur heterologen (Über)expression und zur Antisense-Inhibierung mit dem Ziel, Stoffwechselflüsse in transgenen Pflanzen zu manipulieren sind in Herbers & Sonnewald (1996) TIBTECH 14, 198-205 zusammengefaßt. Ein Beispiel für Ribozyme wurde von Feyter (1996) Mol. Gen. Genet. 250, 329-228 publiziert. Durch Expression eines "hammerhead" Ribozyms gelang es den Autoren, eine TMV-Resistenz in transgenen Tabakpflanzen zu erzeugen. Vielfältige Anwendungsmöglichkeiten von transgenen Pflanzen, die mit Hilfe der erfindungsgemäßen rekombinanten DNA-Moleküle und Vektoren erzeugt werden können, sind auch in TIPTEC Plant Product & Crop Biotechnology, 13 (1995), 312-397 beschrieben.
Die erfindungsgemäßen Vektoren können weitere Funktionseinheiten besitzen, die eine Stabilisierung des Vektors im Wirtsorganismus bewirken, wie einen bakteriellen Replikationsursprung oder die 2-Mikron-DNA zur Stabilisation in *Saccharomyces cerevisiae*. Ferner können "left border"- und "right border"-sequenzen agrobakterieller T-DNA enthalten, sein wodurch eine stabile Integration in das Erbgut von Pflanzen ermöglicht wird. Eine weitere Strategie, Markerfreie transgene Pflanzen zu erzeugen, ist die Verwendung von Sequenz-spezifischen Rekombinasen. Zu diesem Zweck sind z.B. zwei Strategien einsetzbar: (i) Re-Transformation einer Rekombinase exprimierenden Ausgangslinie und Auskreuzung der Rekombinase nach erfolgter Entfernung des Selektionsmarkers, welches mit dem gewünschten Gen assoziiert war. (ii) Co-Transformation mit anschließender Auskreuzung. Voraussetzung für diese Rekombinase-Strategie sind (i) Flankierung des Selektinsmarkers mit Erkennungssequenzen für die Rekombinase und (ii) eine Rekombinase, die in Pflanzen aktiv ist und keine Pflanzen-eigenen Sequenzen zur Rekombination nutzt. Derartige Verfahren kann der Fachmann dem Stand der Technik entnehmen, z.B. RecA (Reiss (1996) Proc. Natl. Acad. Sci. USA 93, 3094-3098), Cre/Iox (Bayley (1992) Plant Mol. Biol. 18, 353-361), FLP/FRT (Lloyd (1994) Mol. Gen. Genet. 242, 653-657), Gin (Maeser (1991) Mol. Gen. Genet. 230, 170-176) und R/RS (Onouchi (1991) Nucl. Acids Res. 19, 6373-6378).

In einer weiteren Ausführungsform betrifft die Erfindung Wirtszellen, die die erfindungsgemäßen rekombinanten DNA-Moleküle oder Vektoren transient oder stabil enthalten. Unter einer Wirtszelle wird ein Organismus verstanden, der in der Lage ist, *in vitro* rekombinierte DNA aufzunehmen und gegebenenfalls die in der erfindungsgemäßen rekombinanten DNA-Molekülen enthaltene DNA-Sequenz zu exprimieren.
Vorzugsweise sind dies prokaryontische oder eukaryontische Zellen. Insbesondere betrifft die Erfindung Pflanzenzellen, die die erfindungsgemäßen Vektorsysteme oder Derivate oder Teile davon enthalten. Diese sind vorzugsweise aufgrund der Aufnahme der erfindungsgemäßen Vektorsysteme, Derivate oder Teile der Vektorsysteme zu einer Synthese von Enzymen befähigt, die die biologische Aktivität einer 2-DOG-6-P Phosphatase besitzen. Vorzugsweise sind die erfindungsgemäßen Zellen dadurch gekennzeichnet, daß das eingeführte erfindungsgemäße rekombinante DNA-Molekül entweder heterolog in Bezug auf die transformierte Zelle ist, d.h. natürlicherweise nicht in diesen Zellen vorkommt, oder an einem anderen Ort im Genom lokalisiert ist als die entsprechende natürlicherweise auftretende Sequenz.

In einer weiteren Ausführungsform betrifft die Erfindung Kits, die ein erfindungsgemäßes rekombinantes DNA Molekül oder einen erfindungsgemäßen Vektor enthalten und gegebenenfalls 2-DOG oder eine zu 2-DOG äquivalente chemische Verbindung. So wurden z.B. von Schmidt (1978) (Eur. J. Biochem. 87, 55-68) 2-deoxy-2-fluoro-D-glucose bzw. mannose als markierte Analoga beschrieben.

Durch die Bereitstellung der erfindungsgemäßen rekombinanten DNA-Moleküle und Vektoren ist es möglich, 2-DOG als Selektionsmittel für die Pflanzentransformation zu benutzen, vorzugsweise zur Selektion transformierter Pflanzen, die von Hochleistungssorten abstammen, für die die im Stand der Technik beschriebenen Selektionsmarker oftmals nur im beschränkten Maße von Nutzen sind.
Die vorliegende Erfindung betrifft somit auch ein Verfahren zur Selektion transformierter Pflanzenzellen, das die folgenden Schritte umfaßt:
(a) Gewinnung von Pflanzenzellen;
(b) Einführung eines erfindinngsgemäßen rekombinanten DNA-Moleküls oder Vektors in diese Pflanzenzellen; und
(c) Selektion der erfolgreich transformierten Pflanzenzellen auf 2-DOG enthaltenden Medien oder auf Medien die eine zu 2-DOG funktionell äquivalente chemische Verbindung enthalten.

Für die Einführung von DNA in eine pflanzliche Wirtszelle stehen eine Vielzahl von Techniken zur Verfügung. Diese Techniken umfassen die Transformation pflanzlicher Zellen mit T-DNA unter Verwendung von Agrobacterium tumefaciens oder Agrobacterium rhizogenes als Transformationsmittel, die Fusion von Protoplasten, die Injektion, die Elektroporation von DNA, die Einbringung von DNA mittels der biolistischen Methode sowie weitere Möglichkeiten.
Bei der Injektion und Elektroporation von DNA in Pflanzenzellen werden an sich keine speziellen Anforderungen an die verwendeten Plasmide gestellt. Es können einfache Plasmide wie z.B. pUC-Derivate verwendet werden. Sollen aber aus derartig transformierten Zellen ganze Pflanzen regeneriert werden, sollte eine selektierbarer Marker vorhanden sein.
Je nach Einführungsmethode gewünschter Gene in die Pflanzenzelle können weitere DNA-Sequenzen erforderlich sein. Werden z.B. für die Transformation der Pflanzenzelle das Ti- oder Ri-Plasmid verwendet, so muß mindestens die rechte Begrenzung, häufig jedoch die rechte und linke Begrenzung der Ti- und Ri-Plasmid T-DNA als Flankenbereich mit den einzuführenden Genen verbunden werden.

Werden für die Transformation Agrobakterien verwendet, muß die einzuführende DNA in spezielle Plasmide cloniert werden, und zwar entweder in einen intermediären Vektor oder in einen binären Vektor. Die intermediären Vektoren können aufgrund von Sequenzen, die homolog zu Sequenzen in der T-DNA sind, durch homologe Rekombination in das Ti- oder Ri-Plasmid der Agrobakterien integriert werden. Dieses enthält außerdem die für den Transfer der T-DNA notwendige vir-Region. Intermediäre Vektoren können nicht in Agrobakterien replizieren. Mittels eines Helferplasmids kann der intermediäre Vektor auf Agrobacterium tumefaciens übertragen werden (Konjugation). Binäre Vektoren können sowohl in E.coli als auch in Agrobakterien replizieren. Sie enthalten ein Selektionsmarker-Gen und einen Linker oder Polylinker, welche von der rechten und linken T-DNA Grenzregion eingerahmt werden. Sie können direkt in die Agrobakterien transformiert werden (Holsters, Mol. Gen. Genet. 163 (1978), 181-187). Das als Wirtszelle dienende Agrobakterium soll ein Plasmid, das eine vir-Region trägt, enthalten. Die vir-Region ist für den Transfer der T-DNA in die Pflanzenzelle notwendig. Zusätzliche T-DNA kann vorhanden sein. Das derartig transformierte Agrobakterium wird zur Transformation von Pflanzenzellen verwendet.
Die Verwendung von T-DNA für die Transformation von Pflanzenzellen ist intensiv untersucht und beschrieben in EP-A-120 516; Hoekema: The Binary Plant Vector System, Offsetdrukkerij Kanters B.V., Alblasserdam (1985), Chapter V, Fraley, Crit. Rev. Plant. Sci., 4, 1-46 und An, EMBO J. 4 (1985), 277-287.
In einer bevorzugten Ausführungsform wird der erfindungsgemäße Vektor in dem erfindungsgemäßen Verfahren mittels Agrobacterium tumefaciens in Pflanzenzellen übertragen.

Für den Transfer der DNA in die Pflanzenzelle können Pflanzen-Explantate zweckmäßigerweise mit Agrobacterium tumefaciens oder Agrobacterium rhizogenes kokultiviert werden. Aus dem infizierten Pflanzenmaterial (z.B. Blattstücke, Stengelsegmente, Wurzeln, aber auch Protoplasten oder Suspensions-kultivierte Pflanzenzellen) können dann in einem geeigneten Medium, welches 2-DOG oder eine funktionell äquivalente Chemikalie zur Selektion transformierter Zellen enthält, wieder ganze Pflanzen regeneriert werden. Die so erhaltenen Pflanzen können dann auf Anwesenheit der eingeführten DNA untersucht werden. Andere Möglichkeiten der Einführung fremder DNA unter Verwendung des biolistischen Verfahrens oder durch Protoplastentransformation sind bekannt (vgl. z.B. Christou (1996) Trends in Plant Science 1, 423-431; Willmitzer, L., 1993 Transgenic plants. In: Biotechnology, A Multi-Volume Comprehensive Treatise (H.J. Rehm, G. Reed, A. Pühler, P. Stadler, eds.), Vol. 2, 627-659, VCH Weinheim-New York-Basel-Cambridge).

Alternative Systeme zur Transformation von monokotylen Pflanzen sind die Transformation mittels des biolistischen Ansatzes, die elektrisch oder chemisch induzierte DNA-Aufnahme in Protoplasten, die Elektroporation von partiell permeabilisierten Zellen, die Makroinjektion von DNA in Blütenstände, die Mikroinjektion von DNA in Mikrosporen und Pro-Embryonen, die DNA-Aufnahme durch keimenden Pollen und die DNA-Aufnahme in Embryonen durch Quellung (zur Übersicht: Potrykus, Physiol. Plant (1990), 269 - 273).
Während die Transformation dikotyler Pflanzen über Ti-Plasmid-Vektorsysteme mit Hilfe von Agrobacterium tumefaciens wohl etabliert ist, weisen neuere Arbeiten darauf hin, daß auch monokotyle Pflanzen der Transformation mittels Agrobacterium basierender Vektoren sehr wohl zugänglich sind (Chan, Plant Mol. Biol. 22 (1993), 491-506; Hiei, Plant J. 6 (1994), 271-282; Bytebier, Proc. Natl. Acad. Sci. USA 84 (1987), 5345 - 5349; Raineri, Bio/Technology 8 (1990), 33 - 38; Gould, Plant Physiol. 95 (1991), 426 - 434; Mooney, Plant, Cell Tiss. & Org. Cult. 25 (1991), 209 - 218; Li, Plant Mol. Biol. 20 (1992), 1037 - 1048).
Drei der oben genannten Transformationssysteme konnten in der Vergangenheit für verschiedene Getreide etabliert werden: die Elektroporation von Gewebe, die Transformation von Protoplasten und der DNA-Transfer durch Partikel-Beschuß in regenerierbare Gewebe und Zellen (zur Übersicht: Jähne, Euphytica 85 (1995), 35 - 44). Die Transformation von Weizen wird in der Literatur verschiedentlich beschrieben (zur Übersicht: Maheshwari, Critical Reviews in Plant Science 14 (2) (1995), 149 - 178).

In einer anderen bevorzugten Ausführungsform wird das erfindungsgemäße rekombinante DNA-Molekül oder der erfindungsgemäße Vektor in dem erfindungsgemäßen Verfahren mittels Particle-Bombardment (biolistische Verfahren) übertragen.

Die vorliegende Erfindung betrifft auch transgene Pflanzenzellen, die ein erfindungsgemäßes rekombinantes DNA-Molekül oder einen erfindungsgemäßen Vektor enthalten oder durch das erfindungsgemäße Verfahren erhalten wurden sowie transgene Pflanzenzellen, die von derartig transformierten Pflanzenzellen abstammen. Derartige Zellen lassen sich von natürlicherweise vorkommenden Pflanzenzellen dadurch unterscheiden, daß sie mindestens ein erfindungsgemäßes rekombinantes DNA-Molekül enthalten, das natürlicherweise in diesen Zellen nicht vorkommt oder dadurch, daß ein solches Molekül an einem Ort im Genom der Zelle integriert vorliegt, in dem es natürlicherweise nicht vorkommt, d.h. in einer anderen genomischen Umgebung.

In einer bevorzugten Ausführungsform enthält die erfindungsgemäße Pflanzenzelle mindestens ein weiteres Fremdgen. Wie bereits in der Einleitung dieser Patentanmeldung erwähnt, ist für die Möglichkeit der Steuerung komplexer Stoffwechselprozesse die Manipulation mehrerer enzymatischer Schritte notwendig und daher die Möglichkeit transgene Pflanzen mehrfach zu transformieren essentiell. Mit Hilfe der erfindinngsgemäßen rekombinanten DNA-Moleküle und Vektoren kann der Fachmann nun auf neue Marker zur Selektion mehrfach transformierter Pflanzenzellen zurückgreifen.

Die transgenen Pflanzenzellen können nach dem Fachmann bekannten Techniken zu ganzen Pflanzen regeneriert werden. Die durch Regeneration der erfindinngsgemäßen transgenen Pflanzenzellen erhältlichen Pflanzen sind ebenfalls Gegenstand der vorliegenden Erfindung. Ferner sind Gegenstand der Erfindung Pflanzen und Pflanzengewebe, die die oben beschriebenen transgenen Pflanzenzellen enthalten. In Abhängigkeit des gewählten Promotors (z.B. 35S CaMV) sind auch die adulten Pflanzen resistent gegen 2-DOG und können durch Gabe dieser Verbindung selektioniert werden. Bei Verwendung von z.B. gewebespezifischen Promotoren entfällt diese Möglichkeit und der Fachmann kann z.B. auf molekularbiologische Methoden wie PCR zurückgreifen, um diese Pflanzen zu identifizieren. Auf der anderen Seite kann der Fachmann natürlich auch, z.B. nach Selbstung oder Rückkreuzung gegen den Elter, Samen solcher Pflanzen auf 2-DOG enthaltene Medien auslegen und anhand der Keimfähigkeit dieser Samen oder Überleben der Pflanzen in einem späteren Stadium der Entwicklung (abhängig vom gewählten Promotor) rückschließen, ob die Pflanzen transgen sind oder nicht. Bei den transgenen Pflanzen kann es sich prinzipiell um Pflanzen jeder beliebigen Pflanzenspezies handeln, d.h. sowohl monokotyle als auch dikotyle Pflanzen. Bevorzugt handelt es sich um Nutzpflanzen wie Weizen, Gerste, Reis, Raps, Erbse, Mais, Zuckerrübe, Zuckerrohr oder Kartoffel.
Die Erfindung betrifft ebenfalls Vermehrungsmaterial und Ernteprodukte der erfindungsgemäßen Pflanzen, beispielsweise Früchte, Samen, Knollen, Wurzelstöcke, Sämlinge, Stecklinge etc.

Wie oben bereits dargelegt, stellt die vorliegende Erfindung rekombinante DNA-Moleküle und Vektoren zur Verfügung, die als Selektionsmarker in Pflanzenzellen zur Selektion transformierter Pflanzenzellen sowie davon abgeleitete transgene Pflanzen, Pflanzenzellen und/oder Gewebe zur Verfügung. So betrifft die vorliegende Erfindung auch die Verwendung eines erfindungsgemäßen rekombinanten DNA-Moleküls oder eines erfindungsgemäßen Vektors zur Herstellung von transgenen Pflanzen, Pflanzenzellen und/oder Gewebe sowie deren Nutzung als selektierbare Marker in pflanzlicher Zell- und Gewebekultur und/oder Pflanzenzüchtung.

**Die Figuren zeigen:**

### Figur 1

Möglicher Mechanismus der Wachstumsinhibierung durch 2-Desoxyglucose (2-DOG) und Aufhebung der toxischen Wirkung durch Expression einer 2-DOG-6-P Phosphatase.

### Figur 2

A. Kultivierung von Tabak- und Kartoffelblattscheiben auf 2-DOG-haltigem MS-Medium. TOB, Nicotiana tabaccum Var. Samsun NN; POT, Solanum tuberosum var. Solara; A, E: Kontrolle MS-Medium ohne Zusatz von 2-Desoxyglukose; B, F: MS-Medium mit 0.05 % 2-DOG; C, G: MS-Medium mit 0.1 % 2-DOG; D, H: MS-Medium mit 0.5 % 2-DOG.
B. Kultivierung von Erbsen-, Raps- und Weizenblattscheiben auf 2-DOG-haltigem MS-Medium. R, Raps; P, Erbse; W, Weizen.
   0.0, 0.1 und 0.5: zugesetzte 2-DOG Konzentration in %.

### Figur 3

Schematische Darstellung der PCR-Amplifikation der 2-DOG-6-P Phosphatase aus Saccharomyces cervisiae Stamm 288C

### Figur 4

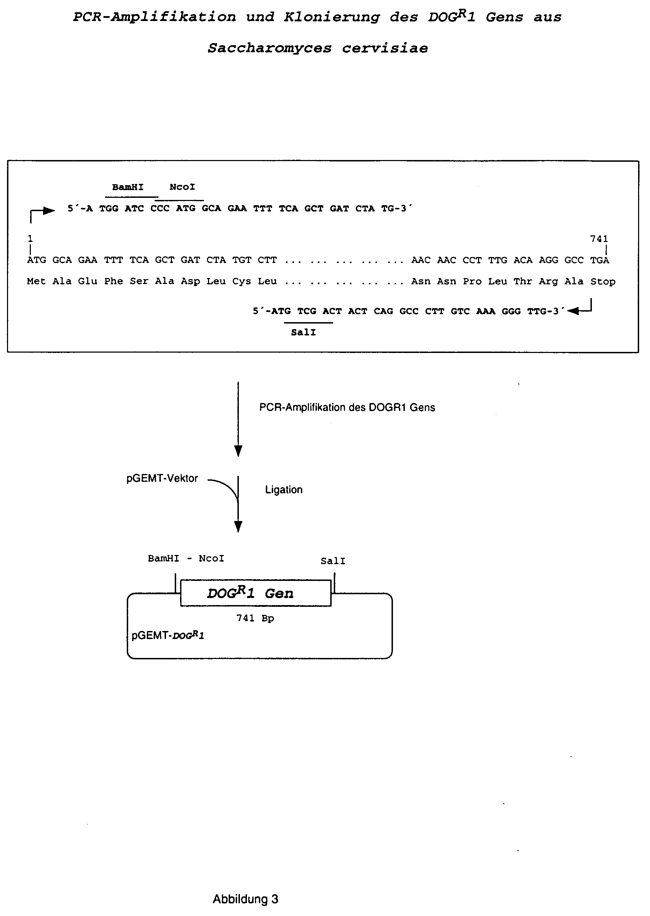

Pflanzliche Expressionskassette zur Überexpression des DOG^{R}1 Gens aus Hefe (*Saccharomyces cervisiae* Stamm S288C) in transgenen Pflanzen
Fragment A (529 bp): Beinhaltet den 35S Promotor des Cauliflower-Mosaik-Virus (CaMV). Es enthält ein Fragment, welches die Nukleotide 6909 bis 7437 des CaMV umfaßt.
Fragment B: DOG^{R}1 Gen aus Hefe, welches als BamHI/SalI Fragment aus dem Plasmid pGEMT-DOG¹R isoliert wurde (s. Abbildung 3).
Fragment C (192 bp): Enthält das Polyadenylierungssignal des Gens 3 der T-DNA des Ti-Plasmids pTiACH5.
Die Fragmente A bis C wurden über EcoRI/HindIII in den binären Vektor BIN19 (Bevan (1984) Nucl. Acid Res. 12, 8711) cloniert.

### Figur 5

Nachweis des DOG^{R}1-Gens mittels PCR-Amplifikation in genomischer DNA der transgenen Tabakpflanzen.
Spuren 1 und 14, DNA-Längenstandard; Spuren 2-10, unabhängige Transformanden; Spur, 11, untransformierte Kontrollpflanze; Spur 12, Positivkontrolle (Plasmid pGEMT-DOG^{R}1); Spur 13, Wasserkontrolle.

### Figur 6

Nachweis der Expression des chimären DOG^{R}1-Gens in 2-DOG resistenten Tabakpflanzen mittels RNA-Analyse.
Northern-Analyse transgener Tabakpflanzen der Linie 35S-DOG. Gesamt RNA wurde aus Tabakblättern isoliert, gelelektrophoretisch aufgetrennt und auf eine Nylonmembran übertragen. Die Hybridisierung erfolgte mit der radioaktiv markierten Kodierregion des DOGR1 Gens. Pro Spur sind 20 µg RNA aufgetragen. Spuren 1-9, unabhängig transformierte Pflanzen der Linie 35S-DOG; Spur 10, untransformierte Kontrolle.

In den Beispielen verwendete Methoden:

### 1. Allgemeine Clonierungsverfahren

Clonierungsverfahren wie z.B.: Restriktionsspaltungen, DNA-Isolierung, Agarose-Gelelektrophorese, Reinigung von DNA-Fragmenten, Transfer von Nukleinsäuren auf Nitrozellulose und Nylon Membranen, Verknüpfen von DNA-Fragmenten, Transformation von E.coli Zellen, Anzucht von Bakterien, Sequenzanalyse rekombinanter DNA wurden nach Sambrook (Cold Spring Harbor Laboratory Press (1989); ISBN 0-87969-309-6) beschrieben durchgeführt. Die Transformation von Agrobacterium tumefaciens wurde entsprechend der Methode von Höfgen und Willmitzer (Nucl. Acid Res. (1988) 16, 9877) ausgeführt. Die Anzucht der Agrobacterien erfolgte in YEB Medium (Vervliet, Gen. Virol. (1975) 26, 33).

### 2. Bakterienstämme

E.coli (XL-1 Blue) Bakterien wurden durch die Firma Stratagene bezogen. Der zur Pflanzentransformation eingesetzte Agrobacterium Stamm (C58C1 mit dem Plasmid pGV 3850kan) wurde von Debleare (1985, Nucl. Acid Res. 13, 4777) beschrieben.

### 3. Analyse von Gesamt-RNA aus pflanzlichen Geweben

Pflanzliche Gesamt-RNA wurde nach der Methode von Logemann (1987, Analytical Biochem. 163, 16) isoliert und in Formaldehyd-haltigen Agarosegelen aufgetrennt (Lehrach (1977) Biochem. 16, 4743). Ein Kappilar-Transfer auf Nylon-Membranen (Gene Screen, NEN) erfolgte in 20 x SSC (1.5 M NaCl, 150 mM Natriumcitrat) über Nacht. Nach zweistündiger Prähybridisierung in Hybridisierungspuffer (500 mM Natriumphosphat (pH 7.2), 7 % SDS, 1 % Rinderserumalbumin, 200 µg Heringssperm DNA, 1 mM EDTA), erfolgte die Hybridisierung mit der radioaktiv-markierten DOG^{R}1-Sonde bei 55°C für 16 Stunden. Als Sonde wurde die DOG^{R}1 Codierregion aus dem Plasmid pGEMT isoliert und mittels High Prime Kit (Boehringer) in Anwesenheit von α-³²P-dCTP radioaktiv markiert. Anschließend wurden die Filter unter folgenden Bedingungen gewaschen: 20 Minuten bei 55°C in 6 x SSC, 0.1 % SDS und 20 Minuten bei 55°C in 4 x SSC, 0.1 % SDS.

### 4. Bestimmung von DOG-6-P in Pflanzenextrakten

Zum Nachweis der 2-DOG-6-P Phosphatase Aktivität in den transgenen Pflanzen wurden Blattscheiben (ca. 100 mg Frischgewicht) in 300 mM 2-DOG Lösung für 24 Stunden im Dunkeln inkubiert. Anschließend wurden die Blattscheiben 1 Minute mit Wasser gewaschen und in flüssigem Stickstoff eingefroren. Zur Extraktion der Metabolite wurde das Pflanzenmaterial in auf Trockenes vorgekühlten Mörsern zu einem feinen Pulver homogenisiert und mit 1.5 ml 16 % (w/v) Trichloressigsäure (TCA) in Diethylether (vorgekühlt auf 4°C) versetzt. Anschließend wurden die Homogenate 15 Minuten auf Trockeneis inkubiert. Durch Zugabe von 0.8 ml 16 % TCA (w/v), 5 mM EGTA wurden die Metabolite gelöst. Nach Transfer der Homogenate in Eppendorfreaktionsgefäße wurden diese 3 Stunden bei 4°C inkubiert. Anschließend wurden die Proben 5 Minuten bei 15 ooo rpm und 4°C in einer Biofuge 15R (Heraeus) zentrifugiert. Die wäßrige Phase wurde in Eppendorfreaktionsgefäße überführt und viermal mit wassergesättigtem Ether gewaschen. Anschließend wurden die Proben mit 5 M KOH/1 M Triethanolamin-Gemisch neutralisiert und für 1 Minute mit Stickstoff begast. Die so vorbereiteten Proben wurden mittels HPLC analysiert. Für die Analyse wurde ein HPLC System der Firma Dionex verwendet, welches mit einer PA-1 (4 x 250 mm) Säule und einem gepulsten elektrochemischen Detektor ausgestattet war. Vor der Injektion wurden die Proben für 2 Minuten bei 13 000 rpm abzentrifugiert. Metabolite wurden anschließend mit einem 50-minütigen konkaven Gradienten (Kurve 9) von 1 mM bis 500 mM Natriumacetat nach 40 Minuten bei 10 mM NaOH und einer Durchflußrate von 1 ml/min. eluiert. Isokratische Elution wurde für weitere 5 Minuten durchgeführt. Zur Identifizierung und Quantifizierung von 2-DOG-6-P wurde als Standard 2-DOG-6-P der Firma Sigma verwendet.

Die Beispiele erläutern die Erfindung.

### Beispiel 1: Nachweis der Toxizität von 2-Desoxyglucose (2-DOG) für Pflanzenzellen

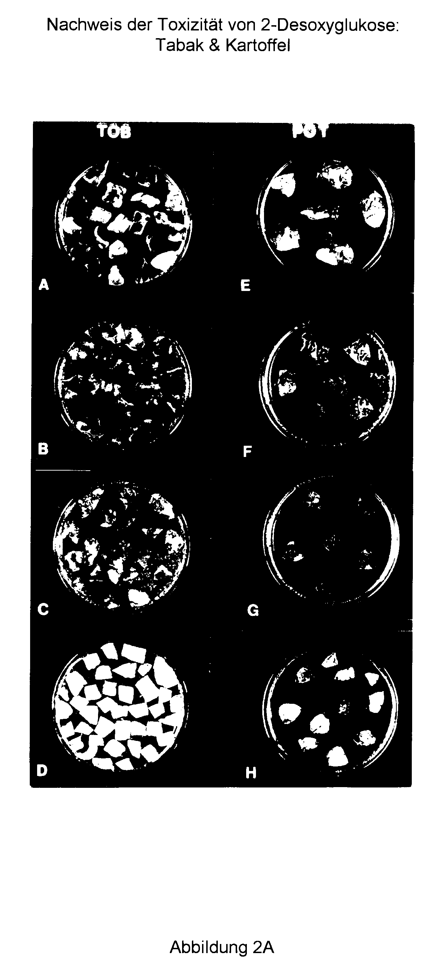

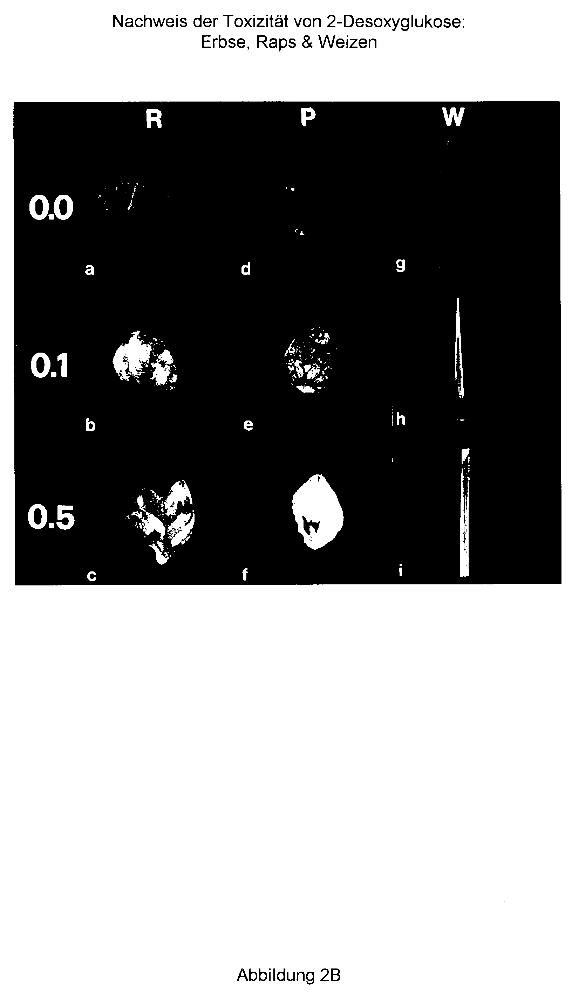

Zum Nachweis der Toxizität von 2-DOG wurden Blattstücke (ca. 1 cm²) von sterilen Tabak- und Kartoffelpflanzen auf Murashige und Skoog-Medium (Murashige & Skoog (1962) Physiol. Plant. 15, 473), dem 0.01 bis 0.5 % 2-DOG zugesetzt wurde, für zwei Wochen kultiviert. Wie in Abbildung 2 gezeigt, führt Zusatz von 2-DOG (Abb. 2, B-D und F-H) zum Absterben der Blattscheiben.

### Beispiel 2: PCR-Amplifikation der 2-DOG-6-P Phosphatase aus Saccharomyces cervisiae Stamm S288C

Die Clonierung der DOG^{R}1 Codierregion erfolgte mittels der Polymerase Kettenreaktion (**P**olymerase **C**hain **R**eaction, PCR). Als Matrizenmaterial diente genomische Hefe DNA, die aus Saccharomyces cervisiae Stamm S288C nach Standardprotokoll isoliert wurde. Die Amplifizierung erfolgte unter Verwendung der spezifischen Primer DOG^{R}1-1 (5'-ATGGATCCCCATGGCAGAATTTTCAGCTGATCTATG-3'; SEQ ID NO:3) und DOG^{R}1-2 (5' ATGTCGACTACTCAGGCCCTTGTCAAAGGGTTG-3'; SEQ ID NO:4), die von einer publizierten Sequenz abgeleitet wurden (Sanz (1994) Yeast 10, 1195-1202). Primer 1 umfaßt die Basen 1 bis 26 und Primer 2 die Basen 720 bis 741 der Codierregion des DOG^{R}1 Gens. Zur Clonierung der amplifizierten DNA in pflanzliche Expressionsvektoren tragen die Primer zusätzlich folgende Restriktionsschnittstellen: Primer 1, BamHI und NcoI; Primer 2, SalI. Die Clonierungsstrategie ist in Abbildung 3 dargestellt. Das PCR Reaktionsgemisch (50µl) enthielt chromosomale Hefe DNA (1µg), Primer 1 und 2 (jeweils 1µg), 10 mM Tris-HCl (pH 8.8 bei 25°C), 3.5 mM MgCl₂, 50 mM KCl, 0.1 % Triton X-100, 200 µM dNTPs (dATP, dCTP, dGTP, dTTP) und 2 Units PrimeZyme DNA Polymerase (Biometra). Vor Zugabe der Polymerase wurde das Gemisch 10 Minuten auf 94°C erhitzt. Die Polymerisierungschritte (60 Zyklen) wurden in einem automatischen "Thermocycler" (Perkin Elmer) nach folgendem Programm durchgeführt: Denaturierung bei 94°C (1 Minute), Anlagerung der Primer bei 40°C (1 Minute), Polymerase-Reaktion bei 72°C (1 Minute). Das erhaltene Fragment wurde in den Vektor pGEMT (Promega Corp., 2800 Woods Hollow Road, Madison, WI 53711-5399, USA) cloniert, wodurch das Plasmid pGEMT-DOGR1 erhalten wurde. Die Identität der amplifizierten DNA wurde mittels Sequenzanalyse verifiziert.

### Beispiel 3: Herstellung von Plasmid p35S-DOG^{R}1

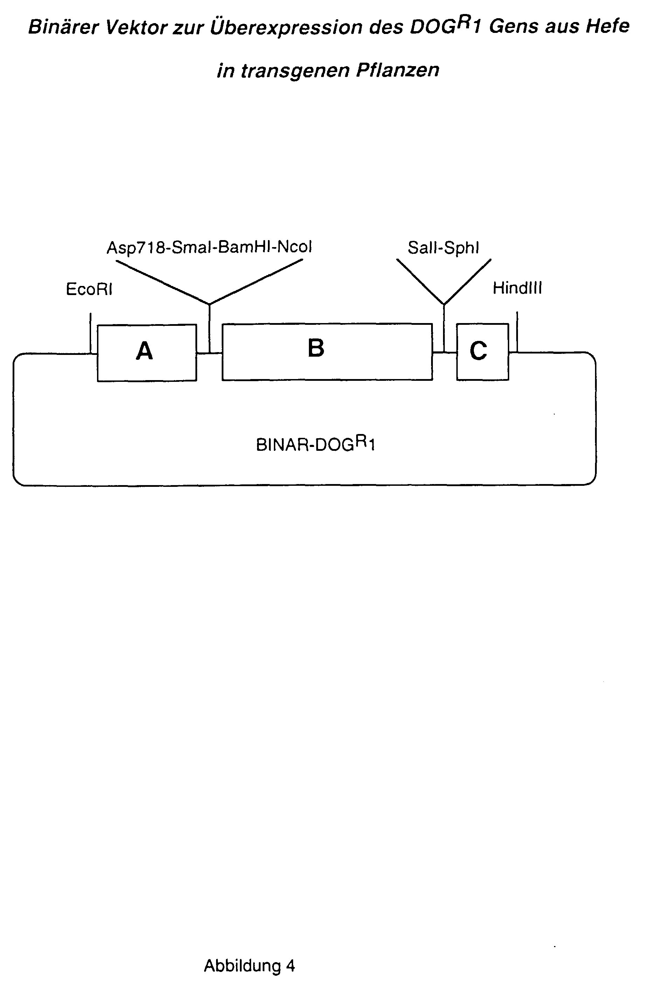

Eine DNA-Sequenz, die für eine 2-DOG-6-P Phosphatase codiert, wurde aus dem Plasmid pGEMT-DOGR1 isoliert und mit dem 35S-Promotor des Cauliflower-Mosaik-Virus, der eine konstitutive Expression in transgenen Pflanzen bewirkt, sowie einem pflanzlichen Transkriptions-Terminationssignal versehen. Das pflanzliche Transkriptions-Terminationssignal beinhaltet das 3'-Ende der Polyadenylierungsstelle des Octopin-Synthase Gens. Das Plasmid p35S-DOG^{R}1 beinhaltet drei Fragmente A, B und C, die in die Schnittstellen für Restriktionsenzyme des Polylinkers von pUC 18 cloniert wurden (s. Abbildung 4).
Das Fragment A beinhaltet den 35S Promotor des Cauliflower-Mosaik-Virus (CaMV). Es enthält ein Fragment, welches die Nukleotide 6909 bis 7437 des CaMV umfaßt (Franck (1980) Cell 21, 285) und wurde als EcoRI-KpnI Fragment aus dem Plasmid pDH51 (Pietrzak (1986) Nucleic. Acid Res. 14, 5857) isoliert und zwischen die EcoRI-KpnI Schnittstellen des Plasmids pUC18 cloniert.

Das Fragment B enthält die DOG^{R}1 Codierregion, die als BamHI-SalI Fragment aus dem Plasmid pGEMT-DOGR1 (s. Abb. 3) isoliert und zwischen die BamHI-SalI Schnittstellen des Polylinkers von pUC18 cloniert wurde.
Das Fragment C enthält das Polyadenylierungssignal des Gens 3 der T-DNA des Ti-Plasmids pTiACH5 (Gielen (1984) EMBO J. 3, 835), Nukleotide 11749 - 11939, welches als PvuII-HindIII Fragment aus dem Plasmid pAGV 40 (Herrera-Estrella (1983) Nature 303, 209) isoliert worden ist und nach Addition von SphI-Linkern an die PvuII-Schnittstelle zwischen SphI-HindIII Schnittstellen des Polylinkers von pUC 18 cloniert war.
Das chimäre Gen wurde anschließend als EcoRI-HindIII Fragment zwischen die EcoRI-HindIII Schnittstellen des Plasmids pBIN19 (Bevan (1984) Nucleic. Acid Res. 12, 8711) cloniert.

### Beispiel 4: Selektion von DOG^{R}1 transformierten Pflanzenzellen und Pflanzenregeneration auf 2-DOG enthaltendem Medium

10 ml einer unter Selektion gewachsenen Übernachtkultur von Agrobacterium tumefaciens wurden abzentrifugiert, der Überstand verworfen, und die Bakterien in gleichen Volumen Antibiotika-freien Medium resuspendiert. In einer sterilen Petrischale wurden Blattscheiben steriler Nicotiana tabacum Var. Samsun NN Pflanzen (ca. 1cm²), denen die Mittelrippe entfernt wurde, in dieser Bakterienkultur gebadet. Anschließend wurden die Blattscheiben in Petrischalen, die MS-Medium mit 2% Saccharose und 0.8% Bacto Agar enthalten, dicht ausgelegt. Nach 2tägiger Inkubation im Dunkeln bei 25^{o}C wurden sie auf MS-Medium übertragen, welches 0.05% 2-DOG, 400 mg/l β-Bactyl, 1 mg/l Benzaminopurin (BAP), 0.2 mg/l Napthylessigsäure (NAA), 1.6 % Glucose und 0.8% Bacto Agar enthielt. Nach Callusbildung wurden die Blattscheiben bis zur Sproßbildung auf MS-Medium übertragen, welches 0.02% 2-DOG, 400 mg/l β-Bactyl, 1 mg/l Benzaminopurin (BAP), 0.2 mg/l Napthylessigsäure (NAA), 1.6 % Glucose und 0.8% Bacto Agar enthielt. Wachsende Sprosse wurden auf hormonfreies MS-Medium mit 200 mg/l β-Bactyl und 2 % Saccharose zur Wurzelbildung überführt. Anschließend wurden bewurzelte Sprosse in Erdkultur überführt und im Gewächshaus kultiviert.

### Beispiel 5: Nachweis des chimären DOG^{R}1-Gens in den transformierten Pflanzen mittels PCR

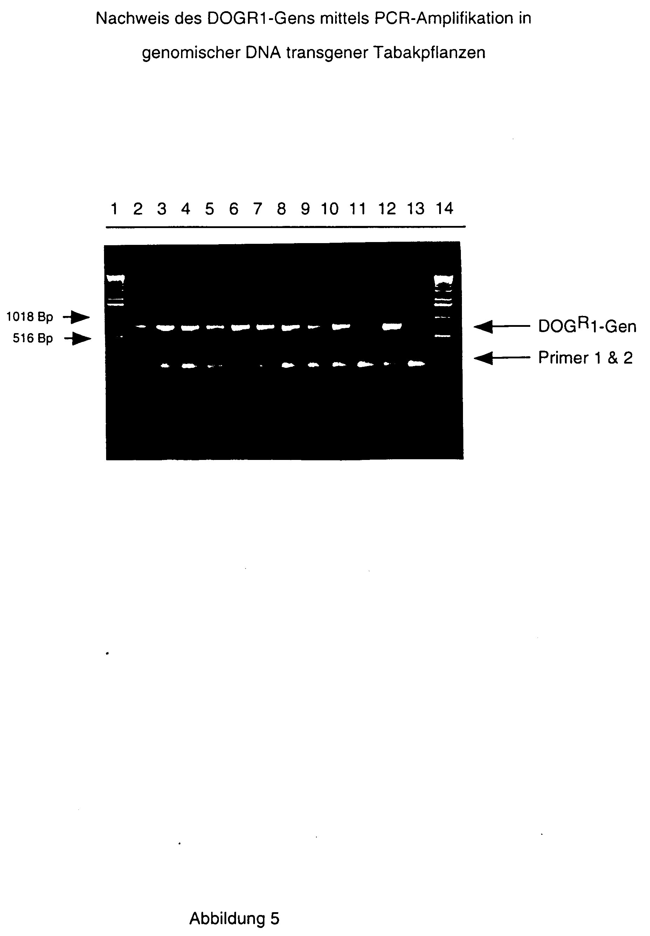

Zum Nachweis der erfolgreichen Transformation wurde genomische DNA der transgenen Tabakpflanzen nach der Methode von Rogers und Bendich (Plant Mol. Biol. (1985) 5, 69) isoliert und das chimäre DOG^{R}1 Gen mittels PCR-Amplifikation nachgewiesen. Das PCR Reaktionsgemisch (50µl) enthielt genomische Tabak DNA (1µg), Primer 1 und 2 (jeweils 1µg), 10 mM Tris-HCl (pH 8.8 bei 25°C), 3.5 mM MgCl₂, 50 mM KCl, 0.1 % Triton X-100, 200 µM dNTPs (dATP, dCTP, dGTP, dTTP) und 2 Units PrimeZyme DNA Polymerase (Biometra). Vor Zugabe der Polymerase wurde das Gemisch 10 Minuten auf 94°C erhitzt. Die Polymerisierungschritte (40 Zyklen) wurden in einem automatischen "Thermocycler" (Perkin Elmer) nach folgendem Programm durchgeführt: Denaturierung bei 94°C (1 Minute), Anlagerung der Primer bei 40°C (1 Minute), Polymerase-Reaktion bei 72°C (1 Minute).
Anschließend wurde ein Aliquot der PCR-Reaktion in einem Agarosegel aufgetrennt. Wie in Abbildung 5 dargestellt, wird unter Verwendung genomischer DNA transgener Pflanzen als Matrize ein ca. 740 Basenpaar großes DNA-Fragment amplifiziert, welches unter Verwendung genomischer DNA von untransformierten Kontrollpflanzen nicht nachweisbar ist.

### Beispiel 6: Nachweis der Expression des chimären DOG^{R}1-Gens in 2-DOG-resistenten Tabakpflanzen mittels RNA-Analyse

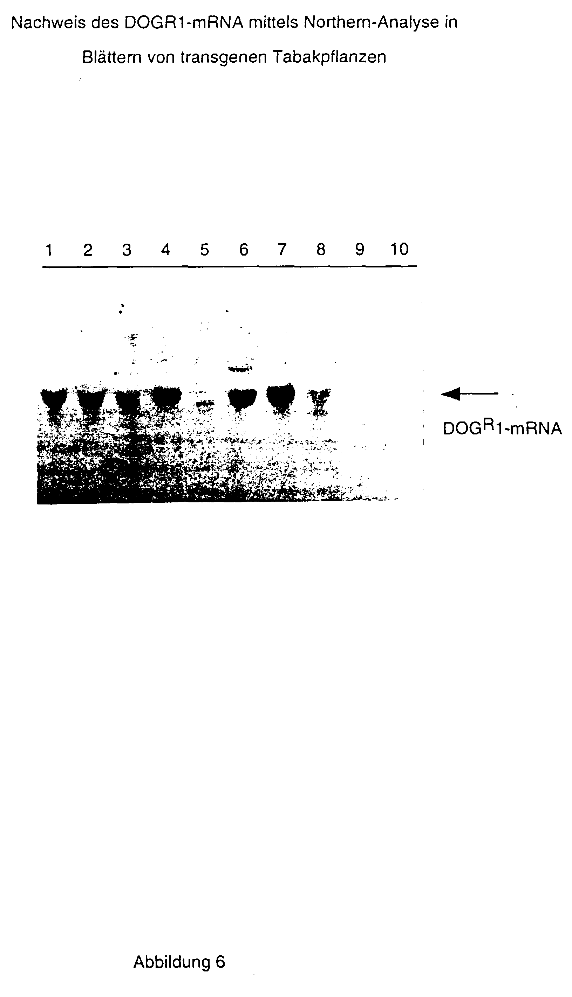

Die Expression der 2-DOG-6-P Phosphatase in den regenerierten Pflanzen wurde mittels RNA-Analysen verifiziert. Hierzu wurden Blattproben der ins Gewächshaus überführten Pflanzen geerntet, Gesamt-RNA extrahiert, elektrophoretisch aufgetrennt, auf Nylonmembranen übertragen und mit der Codierregion des DOG^{R}1 Gens hybridisiert. Wie in Abbildung 6 dargestellt, konnte in den auf 2-DOG regenerierten Tabakpflanzen die 2-DOG-6-P Phosphatase mRNA nachgewiesen werden. Eine Kreuzreaktion mit untransformierten Tabakpflanzen konnte nicht beobachtet werden.

### Beispiel 7: In vivo Nachweis der 2-DOG-6-P Phosphatase Aktivität

Der Nachweis der in vivo Funktionalität der 2-DOG-6-P Phosphatase wurde wie folgt durchgeführt: 0.78 cm² große Blattscheiben von untransformierten Tabakpflanzen und den Transformanden 35S-DOG-3, 4, 8, 9 und 11 wurden 24 Stunden in einer 300 mM 2-DOG Lösung im Dunkeln inkubiert. Zur Detektion des gebildeten 2-DOG-6-P wurden die phosphorylierten Intermediate isoliert und mittels HPLC aufgetrennt. Wie in Tabelle 1 dargestellt, führt die Expression der 2-DOG-6-P Phosphatase zu einer Verminderung der 2-DOG-6-P Akkumulation.

**Tabelle 1**

| Nachweis von 2-DOG-6-P in Blattscheiben von transgenen und untransformierten Tabakpflanzen | | |
|---|---|---|
| Genotyp | [µmol m⁻²] 2-DOG-6-P | [% der Kontrolle] 2-DOG-6-P |
| Kontrolle | 576 ± 40 | 100 |
| 35S-DOG-3 | 170 ± 26 | 31 ± 6.0 |
| 35S-DOG-4 | 223 ± 18 | 40 ± 6.0 |
| 35S-DOG-8 | 103 ± 11 | 18 ± 3.0 |
| 35S-DOG-9 | 250 ± 24 | 45 ± 7.0 |
| 35S-DOG-11 | 283 ± 12 | 50 ± 5.0 |
| Legende: Kontrolle, untransformierte Nicotiana tabacum Var. Samsun NN Pflanze; 35S-DOG, transgene Pflanzen; Die Daten entsprechen den Mittelwerten (n=4) ± Standardabweichung. | | |

## Patentansprüche

1. Rekombinantes DNA-Molekül umfassend
(a) regulatorische Sequenzen eines in Pflanzen aktiven Promotors;
(b) operativ daran gebunden eine DNA-Sequenz, die ein Protein mit der biologischen Aktivität einer 2-Desoxyglucose-6-Phosphat (2-DOG-6-P) Phosphatase codiert; und
(c) operativ daran gebunden regulatorische Sequenzen, die als Transkriptions-Terminations und/oder Polyadenylierungs-signale in Pflanzen dienen können.

2. Rekombinantes DNA-Molekül nach Anspruch 1, wobei die DNA-Sequenz, die ein Protein mit der biologischen Aktivität einer 2-DOG-6-P Phosphatase codiert, ausgewählt ist aus der Gruppe bestehend aus
(a) DNA-Sequenzen, die eine Nucleotid-Sequenz umfassen, die die in SEQ ID NO. 2 angegebene Aminosäuresequenz codieren;
(b) DNA-Sequenzen, die die in SEQ ID NO. 1 angegebene Nucleotidsequenz umfassen;
(c) DNA-Sequenzen umfassend eine Nucleotidsequenz, die mit einem komplementären Strang der Nucleotid-Sequenz von (a) oder (b) hybridisieren;
(d) DNA-Sequenzen umfassend eine Nucleotidsequenz, die zu einer Nucleotidsequenz von (c) degeneriert ist, und
(e) DNA-Sequenzen, die ein Derivat, Analog oder Fragment einer Nucleotidsequenz von (a), (b), (c) oder (d) ist und für ein Protein codiert das 2-DOG-6-P Phosphatase Aktivität besitzt.

3. Rekombinantes DNA-Molekül nach Anspruch 1 oder 2, wobei die DNA-Sequenz aus Hefe stammt.

4. Rekombinantes DNA-Molekül nach einem der Ansprüche 1 bis 3, wobei der Promotor der 35S CaMV Promotor ist.

5. Vektor umfassend ein rekombinantes DNA-Molekül nach einem der Ansprüche 1 bis 4.

6. Vektor nach Anspruch 5, der mindestens ein weiteres rekombinantes DNA-Molekül enthält.

7. Vektor nach Anspruch 6, wobei das weitere rekombinante DNA-Molekül eine DNA-Sequenz enthält, die ein Peptid, Protein, Antisense-, Sense-RNA, virale RNA oder Ribozym codiert.

8. Wirtszelle, enthaltend ein rekombinantes DNA-Molekül nach einem der Ansprüche 1 bis 4 oder einen Vektor nach einem der Ansprüche 5 bis 7.

9. Kit umfassend ein rekombinantes DNA-Molekül nach einem der Ansprüche 1 bis 4 oder einen Vektor nach einem der Ansprüche 5 bis 7 und gegebenenfalls 2-Desoxyglucose oder eine zu 2-Desoxyglucose funktionell äquivalente chemische Verbindung.

10. Verfahren zur Selektion transformierter Pflanzenzellen, das die folgenden Schritte umfaßt:
(a) Gewinnung von Pflanzenzellen;
(b) Einführung eines rekombinanten DNA-Moleküls nach einem der Ansprüche 1 bis 4 oder eines Vektors nach einem der Ansprüche 5 bis 7 in diese Pflanzenzellen; und
(c) Selektion der erfolgreich transformierten Pflanzenzellen auf 2-Desoxyglukose-enthaltenden Medien oder auf Medien die eine zu 2-Desoxyglukose funktionell äquivalente chemische Verbindung enthalten.

11. Verfahren nach Anspruch 10, wobei der Vektor nach einem der Ansprüche 5 bis 7 mittels Agrobacterium tumefaciens in Pflanzenzellen übertragen wird.

12. Verfahren nach Anspruch 10, wobei das rekombinante DNA-Molekül nach einem der Ansprüche 1 bis 4 oder der Vektor nach einem der Ansprüche 5 bis 7 mittels Partikel-Bombardment in Pflanzenzellen übertragen wird.

13. Transgene Pflanzenzelle enthaltend ein rekombinantes DNA-Molekül nach einem der Ansprüche 1 bis 4 oder einen Vektor nach einem der Ansprüche 5 bis 7 oder hergestellt nach dem Verfahren nach einem der Ansprüche 10 bis 12.

14. Pflanzenzelle nach Anspuch 13, die mindestens ein weiteres Fremdgen enthält.

15. Pflanzengewebe umfassend Pflanzenzellen nach Anspruch 13 oder 14 oder hergestellt nach dem Verfahren nach einem der Ansprüche 10 bis 12.

16. Transgene Pflanze enthaltend eine Pflanzenzelle nach Anspruch 13 oder 14 oder hergestellt nach dem Verfahren nach einem der Ansprüche 10 bis 12.

17. Ernteprodukte der Pflanze nach Anspruch 16 umfassend Pflanzenzellen nach Anspruch 13 oder 14.

18. Vermehrungsmaterial der Pflanzen nach Anspruch 16, umfassend Pflanzenzellen nach Anspruch 13 oder 14.

19. Verwendung eines DNA-Moleküls das eine DNA-Sequenz umfaßt wie sie in einem der Ansprüche 1 bis 3 definiert ist, eines rekombinanten DNA-Moleküls nach einem der Ansprüche 1 bis 4 oder eines Vektors nach einem der Ansprüche 5 bis 7 zur Herstellung von transgenen Pflanzen, Pflanzenzellen und/oder Gewebe.

20. Verwendung eines DNA-Moleküls das eine DNA-Sequenz umfaßt wie sie in einem der Ansprüche 1 bis 3 defniert ist, eines rekombinanten DNA-Moleküls nach einem der Ansprüche 1 bis 4 oder eines Vektors nach einem der Ansprüche 5 bis 7 als selektierbarer Marker in pflanzlicher Zell- und Gewebekultur und/oder Pflanzenzüchtung.


SEQUENZPROTOKOLL

(1) ALLGEMEINE ANGABEN:

(i) ANMELDER:
(A) NAME: IPK Gatersleben
(B) STRASSE: Corrensstr. 3
(C) ORT: Gatersleben
(E) LAND: Germany
(F) POSTLEITZAHL: 06466

(ii) BEZEICHNUNG DER ERFINDUNG: 2-DOG-6-P Phosphatase DNA-Sequenzen als
Selektionsmarker in Pflanzen

(iii) ANZAHL DER SEQUENZEN: 4

(iv) COMPUTER-LESBARE FASSUNG:
(A) DATENTRÄGER: Floppy disk
(B) COMPUTER: IBM PC compatible
(C) BETRIEBSSYSTEM: PC-DOS/MS-DOS
(D) SOFTWARE: PatentIn Release #1.0, Version #1.30 (EPA)

(2) ANGABEN ZU SEQ ID NO: 1:

(i) SEQUENZKENNZEICHEN:
(A) LÄNGE: 758 Basenpaare
(B) ART: Nucleotid
(C) STRANGFORM: Einzelstrang
(D) TOPOLOGIE: linear

(ii) ART DES MOLEKÜLS: cDNA

(iii) HYPOTHETISCH: NEIN

(iv) ANTISENSE: NEIN

(vi) URSPRÜNGLICHE HERKUNFT:
(A) ORGANISMUS: Saccharomyces cerevisiae

(ix) MERKMAL:
(A) NAME/SCHLÜSSEL: CDS
(B) LAGE:9..746

(xi) SEQUENZBESCHREIBUNG: SEQ ID NO: 1:

GGATCCCC ATG GCA GAA TTT TCA GCT GAT CTA TGT CTT TTT GAC CTA GAT 50
Met Ala Glu Phe Ser Ala Asp Leu Cys Leu Phe Asp Leu Asp
1               5                   10

GGT ACC ATA GTG AGT ACA ACA GTG GCC GCA GAG AAA GCA TGG ACC AAG 98
Gly Thr Ile Val Ser Thr Thr Val Ala Ala Glu Lys Ala Trp Thr Lys
15                  20                  25                  30
TTG TGT TAC GAA TAC GGT GTT GAT CCT TCC GAG TTA TTT AAG CAT TCT 146
Leu Cys Tyr Glu Tyr Gly Val Asp Pro Ser Glu Leu Phe Lys His Ser

                    35                  40                  45

CAT GGT GCA AGA ACA CAA GAG GTT TTG AGA AGG TTT TTC CCT AAA TTG        194
His Gly Ala Arg Thr Gln Glu Val Leu Arg Arg Phe Phe Pro Lys Leu
                50                  55                  60

GAT GAT ACA GAC AAT AAA GGT GTT CTT GCT CTA GAA AAA GAT ATT GCC        242
Asp Asp Thr Asp Asn Lys Gly Val Leu Ala Leu Glu Lys Asp Ile Ala
            65                  70                  75

CAT AGT TAC TTG GAT ACA GTA AGC CTT ATT CCT GGT GCA GAG AAC TTA        290
His Ser Tyr Leu Asp Thr Val Ser Leu Ile Pro Gly Ala Glu Asn Leu
        80                  85                  90

CTG TTA TCG TTA GAT GTA GAT ACT GAG ACT CAA AAA AAG TTA CCT GAA        338
Leu Leu Ser Leu Asp Val Asp Thr Glu Thr Gln Lys Lys Leu Pro Glu
 95                 100                 105                 110

AGG AAA TGG GCT ATC GTT ACC TCT GGT TCT CCA TAT TTG GCA TTT TCA        386
Arg Lys Trp Ala Ile Val Thr Ser Gly Ser Pro Tyr Leu Ala Phe Ser
                115                 120                 125

TGG TTC GAG ACA ATA TTG AAA AAT GTT GGA AAG CCC AAA GTT TTC ATT        434
Trp Phe Glu Thr Ile Leu Lys Asn Val Gly Lys Pro Lys Val Phe Ile
            130                 135                 140

ACT GGG TTT GAC GTG AAG AAC GGT AAG CCT GAT CCC GAG GGT TAT TCA        482
Thr Gly Phe Asp Val Lys Asn Gly Lys Pro Asp Pro Glu Gly Tyr Ser
        145                 150                 155

AGA GCT CGT GAT TTA TTG CGT CAA GAT TTG CAA TTA ACT GGT AAA CAG        530
Arg Ala Arg Asp Leu Leu Arg Gln Asp Leu Gln Leu Thr Gly Lys Gln
    160                 165                 170

GAT CTG AAG TAT GTT GTC TTC GAA GAT GCA CCC GTG GGC ATA AAG GCC        578
Asp Leu Lys Tyr Val Val Phe Glu Asp Ala Pro Val Gly Ile Lys Ala
175                 180                 185                 190

GGC AAA GCA ATG GGC GCC ATT ACT GTG GGT ATA ACA TCC TCG TAT GAC        626
Gly Lys Ala Met Gly Ala Ile Thr Val Gly Ile Thr Ser Ser Tyr Asp
                195                 200                 205

AAG AGC GTT TTA TTT GAC GCA GGA GCA GAT TAT GTA GTC TGT GAT TTG        674
Lys Ser Val Leu Phe Asp Ala Gly Ala Asp Tyr Val Val Cys Asp Leu
            210                 215                 220

ACA CAG GTT TCC GTG GTT AAG AAC AAT GAA AAC GGT ATT GTC ATC CAG        722
Thr Gln Val Ser Val Val Lys Asn Asn Glu Asn Gly Ile Val Ile Gln
        225                 230                 235

GTA AAC AAC CCT TTG ACA AGG GCC TGAGTAGTCG AC                          758
Val Asn Asn Pro Leu Thr Arg Ala
    240                 245

(2) ANGABEN ZU SEQ ID NO: 2:

(i) SEQUENZKENNZEICHEN:
(A) LÄNGE: 246 Aminosäuren
(B) ART: Aminosäure
(D) TOPOLOGIE: linear

(ii) ART DES MOLEKÜLS: Protein
(xi) SEQUENZBESCHREIBUNG: SEQ ID NO: 2:

Met Ala Glu Phe Ser Ala Asp Leu Cys Leu Phe Asp Leu Asp Gly Thr
1               5                   10                  15

Ile Val Ser Thr Thr Val Ala Ala Glu Lys Ala Trp Thr Lys Leu Cys
            20                  25                  30

Tyr Glu Tyr Gly Val Asp Pro Ser Glu Leu Phe Lys His Ser His Gly
        35                  40                  45

Ala Arg Thr Gln Glu Val Leu Arg Arg Phe Phe Pro Lys Leu Asp Asp
    50                  55                  60

Thr Asp Asn Lys Gly Val Leu Ala Leu Glu Lys Asp Ile Ala His Ser
65                  70                  75                  80

Tyr Leu Asp Thr Val Ser Leu Ile Pro Gly Ala Glu Asn Leu Leu Leu
                85                  90                  95

Ser Leu Asp Val Asp Thr Glu Thr Gln Lys Lys Leu Pro Glu Arg Lys
            100                 105                 110

Trp Ala Ile Val Thr Ser Gly Ser Pro Tyr Leu Ala Phe Ser Trp Phe
        115                 120                 125

Glu Thr Ile Leu Lys Asn Val Gly Lys Pro Lys Val Phe Ile Thr Gly
    130                 135                 140

Phe Asp Val Lys Asn Gly Lys Pro Asp Pro Glu Gly Tyr Ser Arg Ala
145                 150                 155                 160

Arg Asp Leu Leu Arg Gln Asp Leu Gln Leu Thr Gly Lys Gln Asp Leu
                165                 170                 175

Lys Tyr Val Val Phe Glu Asp Ala Pro Val Gly Ile Lys Ala Gly Lys
            180                 185                 190

Ala Met Gly Ala Ile Thr Val Gly Ile Thr Ser Ser Tyr Asp Lys Ser
        195                 200                 205

Val Leu Phe Asp Ala Gly Ala Asp Tyr Val Val Cys Asp Leu Thr Gln
    210                 215                 220

Val Ser Val Val Lys Asn Asn Glu Asn Gly Ile Val Ile Gln Val Asn
225                 230                 235                 240

Asn Pro Leu Thr Arg Ala
                245

(2) ANGABEN ZU SEQ ID NO: 3:

(i) SEQUENZKENNZEICHEN:
(A) LÄNGE: 36 Basenpaare
(B) ART: Nucleotid
(C) STRANGFORM: Einzelstrang
(D) TOPOLOGIE: linear

(ii) ART DES MOLEKÜLS: Sonstige Nucleinsäure

(iii) HYPOTHETISCH: JA

(iv) ANTISENSE: NEIN

(xi) SEQUENZBESCHREIBUNG: SEQ ID NO: 3:

ATGGATCCCC ATGGCAGAAT TTTCAGCTGA TCTATG 36

(2) ANGABEN ZU SEQ ID NO: 4:

(i) SEQUENZKENNZEICHEN:
(A) LÄNGE: 33 Basenpaare
(B) ART: Nucleotid
(C) STRANGFORM: Einzelstrang
(D) TOPOLOGIE: linear

(ii) ART DES MOLEKÜLS: Sonstige Nucleinsäure

(iii) HYPOTHETISCH: JA

(iv) ANTISENSE: NEIN
(xi) SEQUENZBESCHREIBUNG: SEQ ID NO: 4:

ATGTCGACTA CTCAGGCCCT TGTCAAAGGG TTG 33